# EUROPEAN PATENT APPLICATION

(11) **EP 0 701 001 A2**
(43) Date of publication of application: **13.03.1996**
(21) Application number: 95114067.2
(22) Date of filing: 07.09.1995
(51) Int. Cl.: C12Q 1/68

(54) **DNA separating, fractionating and analyzing method and system therefor**

(30) Priority: 07.09.1994 JP 214055/94
(71) Applicant: HITACHI, LTD., Chiyoda-ku, Tokyo 101 (JP)
(72) Inventor: Kambara, Hideki, Hachiouji-shi, Tokyo 192 (JP); Okano, Kazunori, Shiki-shi, Saitama-ken 353 (JP)
(74) Representative: Strehl Schübel-Hopf Groening & Partner

(57) **Abstract**

A long DNA is digested into small fragments to be sequenced without subcloning using a small library of 16 primers. Each DNA fragment can be isolated by the combined technique of gel electrophoresis separation and hybridization coupled with DNA polymerase reaction, which does not use subcloning, therefore, is good for full automation of the process. The present invention comprises: a process of creating a plurality of DNA fragments by digesting the target DNA with restriction enzyme or the like and introducing the oligomer having known sequence to their termini through ligation reaction or terminal base addition reaction by terminal transferase; a process of preparing the DNA probe comprising the oligomers which include the known sequence and the sequence for selecting fragments where 1 to 3 bases of the 3' terminal side of the DNA probe can take every sequences but they are held separately for each sequence selecting species; a process of hybridizing the DNA fragment with the DNA probe on the solid; a process of stabilizing hybrid by extending only the DNA probes in the hybrid for which the selective sequence of two bases (1-3 bases) at 3' terminus of DNA probe is perfectly complementary to the DNA fragments; and a process of removing the DNA fragments hybridized with the DNA probes which were not subjected to complementary strand extension, and of selecting and fractionating the specific DNA fragment. Addition of a process of analyzing the DNA sequence of the DNA fragments separated and fractionated by this separation and fractionation method provides a DNA analysis method according to the present invention. Whenever required, the separation and fractionation process by gel electrophoresis may be used in combination. Said DNA separating, fractionating and analyzing method according to the present invention uses the DNA separation and fractionation system comprising the vessel incorporating the solid immobilizing said DNA probes and a robot system comprising a means for loading substances required for said vessel, a means for temperature control, a sample transfer means and a means for fractionating the separated target DNA fragment.

## Description

### BACKGROUND OF THE INVENTION

The present invention relates to the sample preparation method, DNA analysis method and system thereof for DNA separating, fractionating and analyzing method used in DNA sequencing or DNA analysis.

For long DNA sequencing according to the conventional method, the target DNA is cloned in the vector such as plasmid, thereby increasing the number of DNA copies by culture for the intended use. To sequence said DNA (2-kilo to 6-kilo bases in the present context), (1) the DNA is digested by restriction enzyme into smaller fragments, (2) they are infected into the colibacilli after re-cloning (subcloning), and (3) said colibacilli are cultured in colony. Then DNA fragments is fractionated by colony selection. The colibacilli in the colony selected are propagated by culture and the DNA is extracted to get many copies of the target DNA embedded in the plasmid. Namely, the DNA is digested by restriction enzyme to purify, fractionate and amplify DNA fragments by subcloning and the subsequent colony culture. The colibacilli cultured on agar form colonies, as described above. Each colony contains colibacilli provided with plasmid containing a single species of DNA fragments. Colibacilli are picked up from each colony and are cultured in a tube to increase the number of copies. Then the DNA is extracted to be used as a sequencing sample. To analyze the various DNA fragments, the DNA fragments obtained from various species of colonies are analyzed. Since there are cases of repeated analysis of the colonies containing the same DNA fragments, analysis is redundant and often made of the number of the colonies about four times that of DNA fragment species on an average.

DNA preparation using such subcloning allows the DNA fragments to be separated and amplified in one process. Despite this advantage, however, it had a disadvantage of taking much time and labor, and was not suited to automation. Furthermore, it had anther disadvantage of having to make repeated analyses of the same DNA fragments, since it is not possible to know in advance what DNA fragment species are contained in the colibacillus of each colony.

### SUMMARY OF THE INVENTION

The object of the present invention is to solve the said problems of the prior arts and to provide a DNA fractionation, proliferation and analysis method without using the cloning and culture, thereby replacing the subcloning and culture processes which are time-consuming and unfit for automation.

To attain the said objective, the present invention causes hybridization between the DNA fragment and the DNA probe sets with 1 to 3 bases of the 3' terminal side having sequence selecting DNA fragments; thus, the DNA probe which can be used as primer for sequencing each DNA fragment is determined by whether the probe DNA strand extension reaction takes place or not. The present invention comprises the method of fractionating the DNA using said method. The term DNA used in this context is a little different from the conventional definition. The sequences of the majority of two or more DNA probes are the same, with only the 1 to 3 bases of the 3' terminal side being different. The purpose is to observe presence or absence of complementary strand extension, not presence or absence of hybridization. The DNA fragments are propagated by PCR (polymerase chain reaction), whenever required, and separation and fractionation according to the length of the DNA fragment by gel electrophoresis are performed in parallel to the said newly developed method, thereby performing DNA analysis.

As discussed above, in the cloning or subcloning process, various species of DNA fragments are fractionated to increase the number of copies. If two processes of fractionation and amplification can be replaced by a handy process fitted for automation, it will be very helpful for large-scale DNA sequencing. Use of the PCR method is already spread as a means of increasing the number of DNA fragment copies. So the problem will be solved if various DNA fragments are fractionated or DNA sequencing is performed under mixed condition. Already disclosed means to fractionate the DNA fragment include gel electrophoresis separation or the method of immobilizing oligomer on the solid surface to use the presence or absence of hybridization. Gel electrophoresis, however, fails to separate the DNA fragments having almost the same length or complementary strands. Furthermore, when hybridization is used, identification is made by whether or not the specific sequence is possessed by some of the relevant DNA fragment groups; therefore, not all sequences can fractionate each fragment of an unknown fragment group. To solve this problem, an system is proposed wherein each fragment is fractionated and analyzed by using several bases at terminal of each fragment as an index. Namely, when the terminal of each fragment is digested by the restrictive enzyme, there is the recognition sequence of the enzyme at the cutting sites of all the fragments, but the sequences of 1 to 3 bases adjacent thereto are expected to differ according to each fragment; this is used for selection in said proposed method. Or when this DNA fragment is digested at random positions by supersonic wave, etc., attention is paid to the fragment having the special sequence of three to four bases at terminal voluntarily chosen, in place of restriction enzyme recognizing sequence (When GACT is selected as four bases, it appears at the rate of one out of 256 bases); then the fragment having the terminal sequence appearing at this constant frequency is identified and fractionated by using the difference of the 1- to 3-base sequence adjacent thereto, according to an alternative method. To realize this, the present invention uses gel electrophoresis to separate the fragments roughly and fractionate them. To identify the DNA fragments in each fraction, use is made of the difference of the terminal several mers, 4 to 7 mers in many cases. In these 4 to 7 mers, 2 to 4 mers are the recognition sequence of the restriction enzyme or the sequence appearing at specified intervals, while the remaining 1 to 3 mers at the 3' terminus are used for selection. A successful result cannot be achieved by an attempt made to prepare the DNA probe having the sequence complementary to this 4- to 7-mer portion, and to fractionate it according to whether or not the hybridization is perfect or not. This is because of the following: Firstly, the 4- to 7-mer hybridization fails to provide a stable formation of the hybrid. Secondly, accurate fractionation excluding one base mismatch cannot be achieved by hybridization base. Consequently, unknown DNA fragments cannot be identified or separated by using the simple DNA hybridization method having been used so far. According to this proposal, in order to ensure stable hybridization, the DNA oligomer of known sequence is added to the 3' terminal of the DNA fragments to prolong the hybridization area. And the DNA probe for selection having the selected sequence at it's 3' terminal is hybridized with the 3' terminal of the fragments, and the DNA fragment is fractionated not merely according to whether hybridization takes place or not, but also according to whether or not the hybridized DNA probe causes extension of the DNA strand through DNA complementary strand extension reaction. It uses the following fact: Whether complementary strand extension takes place or not depends on whether 1 to 3 bases of the 3' terminal achieves a perfect matching or not. The DNA analysis method using the DNA probe discussed above includes; 1) digestion of target DNA, 2) fractionation of the digested DNA fragments after gel electrophoresis, 3) modification of the DNA fragments by adding oligomer to the 3' termini, 4) hybridization of DNA probes with the fragments, 5) the extension of DNA probes by polymerase reactions which occur when the 3' termini of the probes completely match with the fragments, 6) determination of the sequencing primers by measuring extended primers, and /or fractionation of the DNA fragments with the extended DNA probes, and 7) DNA sequencing from the mixture of the DNA fragments using the determined primer or DNA sequencing of the fragment fractionated with the extended DNA probe. The keys of the process are (5) and (6). For example, when the selected sequence is 2 mers, there are 16 species of the DNA probes. The DNA samples are divided into the 16 equal fractions, and the DNA probes different from each other are inserted in the fractions to provide complementary strand extension. All the sequences are common except for the two bases of 3' terminal; hybridization occurs with any fragment. However, an effective complementary strand extension takes place only when two bases of the terminal are perfectly hybridized. The priming ability of DNA probes is investigated with fluorophore labeled DNA probes. The length of product resulting from reaction is analyzed by gel electrophoresis. Thus, checking the length according to migration speed will identify the DNA probe which functions as the sequencing primer of the DNA fragment, as well as the approximate length of the DNA fragment. When the DNA probe is labeled by biotin instead, the DNA fragment can be picked up by the magnetic bead with streptavidin immobilized. If the temperature is set to 80 to 85 degrees Celsius in this case, the DNA fragment which is hybridized with the DNA probe without complementary strand extension is unstable to leave from the DNA probe and removed. The DNA fragment which is hybridized with the extended DNA probe does not leave from the extended DNA probe and therefore, it can be fractionated. To make this process more effective, 16 cells are provided on the solid surface, and 16 species of DNA probes are immobilized to these cells respectively to form a chip, which is used for the present purpose. Needless to say, it is also possible to immobilize the DNA probes to a bead instead of the chip and to enclose them into the cells which are different according to the species of the held DNA. A different vessel may also be used. The DNA fragment mixture is put into a vessel containing the chip, and hybridization is caused between the immobilized oligomers. This process is followed by complementary strand extension of DNA probes using the DNA polymerase. When the selective sequence comprising two bases of the 3' terminal of the DNA probe is complementary to the sequence of the hybridized DNA, complementary strand extension occurs to ensure a greater stability of the hybridization. Then the temperature is made to rise to 80 to 85 degrees Celsius; then the DNA fragment attached to the DNA probe which is not subjected to complementary strand extension leaves from the DNA probe and is washed away by solvent; then it is removed. This results in holding only the DNA fragments with the extended DNA probe. By identifying the species of these DNA probes, it is possible to select the primer for determing the sequence of the specific fragment from the DNA fragment mixture. It is also possible to fractionate the DNA fragment trapped in the cell according to each species to use it as a sequencing template. The above has mainly discussed the DNA analysis. Needles to say, it is also an effective method of fractionating only the DNA which is hybridized with the DNA probe which is perfectly matched due to the DNA strand extension.

Namely, the present invention makes it possible to get the information for analysis by identifying the DNA fragments according to the terminal sequence through the use of the DNA probe hybridization and its ensuing complementary strand extension, and to fractionate the DNA fragments, thereby providing a method for sequencing the DNA having unknown sequence. It is also possible to sequence the DNA fragments separated and fractionated according to the said procedure, after their copies have been amplified whenever as required. DNA fragments as start-up substances for the separation and fractionation procedure described above, or DNA analysis can be prepared in any treatment method; for example, ultrasonic digestion or enzymatic DNA digestion with restriction enzyme. The restriction enzyme utilized for this purpose includes the HhaI, Hind III, Sau3A1, Not1 and ECORI. One or several of them are selected properly in conformity to the relevant the DNA sequence. Also, digestion by exonuclease or endonuclease, digestion by supersonic wave or digestion by chemical reaction can be used to produce the DNA fragments.

Furthermore, the DNA separation and fractionation method may use the following method where amplification in the number of copies of the DNA fragments is added to the method of using the said processes 1) to 7). Namely, this DNA separation and fractionation has at least the following processes:
I) A process of combining the oligomer having the known sequence with a plurality of DNA fragments;
II) a process of preparing the chip wherein the DNA probe comprising the oligomer which includes the sequence complementary to the known sequence portion and where 1 to 3 bases of the 3' terminal side is sequence-select is immobilized on the solid surface. for each species of the sequence selecting DNA probe, or a process of holding the probe in a compartmentalized vessel; and
III) a process of amplifying the number of copies of the said DNA fragments by complementary strand extension with enzyme by using the compartmentalized and held DNA probe obtained in Process II) and the primer which has a sequence complementary to the sequence common to said known sequence and which is hybridized with the terminals of all fragments, and of separating the DNA fragments for each species to perform separation and fractionation on the solid surface.

The length of the base of the known sequence portion of the DNA probe having the sequence complementary to the said known sequence should meet the requirement of forming double strands by hybridization. It is preferred to be 10 to 30 mer, and should be 16 to 20 mer more preferably. It should be noted that, when poly A (polyadenylic acid) is to be added by terminal transferase, the additional stand length may reach about 100 mer. In such cases, the length of the DNA probe to select the DNA fragments should be 10 to 30 mer. Unstable hybridization will result if the length is smaller than 10 mer. If it is greater than 30 mer, hybridization tends to occur even to the portion which is not perfectly complementary; this is not preferred. About 18 mer (normally used size) is appropriate for the primer. Note that labeling the oligomer added to the DNA fragment is more convenient for subsequent separation. Fluorophore, chemiluminescence inducing substance or radioactive substance are used as the label.

The said DNA analysis, separation and fractionation methods according to the present invention can be made more effective by a combined use of separation and fractionation according to the length of the DNA fragment based on gel electrophoresis.

Separation and fractionation by gel electrophoresis are carried out according to the method of separating the DNA band by using the gel plate having a polyacrylamide concentration of 6% (in the present specification, the gel concentration is expressed in terms of percentage of weight/volume (g/ml) of total monomer concentration), or the method of fractionation by eluting the DNA into the solution from the tube gel terminal (e.g. disclosed in U.S. Patent 5,277,780).

The following describes the way of preparing the solid holding the oligomer according to the present invention: To immobilize the oligomer to the solid surface, it is possible to use the method disclosed in K. Beattie et al; Clinical Chem., Vol. 39 (1993), pp. 719 - 722. Or according to the method described in M. Mitsuhashi et al; Nature, Vol. 357 (1992), pp. 519-520, the target DNA probe is formed on to the 3' terminal side of the poly T (polythymidylic acid) by complementary strand extension, after hybridizing with said poly T the oligomer having the sequence complementary to both the poly A and target oligomer sequence by using the plastic surface holding the poly T. Plastics, quartz plate, silicon wafer, etc. are used as the solid base holding this DNA probe.

As discussed above, the selecting sequence in the DNA probe for DNA fragment selections should be 1 to 3 bases long. Needless to say, the selecting sequence can be 4 to 6 bases long through repetition of this process twice or more, but must be 1 to 3 bases per operation. One of the basic principles according to the present invention is that, the DNA probe free or immobilized to the solid carry out complementary strand extension (said process 5 or process III) to form stable hybrids with the target DNA which can be identified from other DNA fragments by the hybrid formation, only when the selecting sequence on the 3' terminal side of the DNA probe for DNA selection is accurately hybridized with the DNA fragment. The complementary strand extension capability in this case heavily depends on whether or not the sequence of 1 to 3 bases at the 3' terminus is perfectly complementary with the DNA fragment sequence. It is an essential requirement of the present invention that at least one arbitrary base for selection is provided on the 3' terminal side of the selecting DNA probe. Furthermore, when the selecting sequence on the 3' terminal side of the DNA probe for selection is four or more bases long, complementary strand extension will proceed despite mismatching of one base at this portion, resulting in a failure to identify the perfectly matched DNA probe from mismatched DNA probe; this is not preferred, of course.

Complementary strand extension by DNA polymerase is carried out at the reaction temperature of 70 to 75 degrees Celsius, with the mixture of the 2 units of Taq as DNA polymerase and dNTP (deoxynucleotide triphospate) as DNA polymerase reaction mixture added into a vessel holding the chip hybridized with the target DNA fragment. If the reaction temperature is lower than 70 degrees Celsius, reaction efficiency will deteriorate; this is not preferable. Activity is important for such enzyme as Tag, and its quantity (e.g. defined by the amount of enzyme to decompose or synthesize a specified amount of DNA in a specified time) is expressed in terms of the unit.

The target DNA fragment of 0.1 to 1 p mol (10¹¹ to 10¹² molecules) is normally used for DNA sequencing. Accordingly, if the amount of the target DNA fragment is not enough for sequencing, the number of DNA fragment copies are amplified by enzyme as shown in said process III. This can be performed under the condition of PCR (polymerase chain reaction) normally adopted.

Fluorescence detection type automated DNA sequencer, namely, fluorescence detection type electrophoresis apparatus is used to analyze the DNA sequence, and the base sequence is determined according to its standard protocol.

The PCR is effective in increasing the number of DNA fragment copies, while gel electrophoresis is effective in separating the DNA fragments according to the length and in grouping them. The oligomer of known sequence is attached to both ends of the DNA fragment fractionated by gel electrophoresis by ligation or by addition of dATP by terminal transferase. This is intended to add an area to the fragment for ensuring stable hybridization of the primer. The DNA probe comprising this sequence and the sequence complementary to the known sequence such as sequence of the digested portion by restriction enzyme and which has, as selecting sequences, two sequence selecting bases on the 3' terminal side, is compartmentalized for each terminal sequence, with the 5' terminal side of the probe immobilized into the vessel which is prepared. The solution containing the DNA fragment is put into the vessel and is made to hybridize with the immobilized probe. The DNA probe is extended by the DNA polymerase. The strand extension occurs only when the 3' terminal sequence of the probe is complementary to DNA fragment, and the stability of the hybrid is increased.

When the temperature is raised to 80 to 85 Centi-degrees, the hybrid of the nonextended primer and DNA fragment dissociates. On the other hand, the hybrid of the extended primer and DNA fragment does not dissociate and the DNA fragment remains on the solid surface. DNA fragments left from the DNA probe are removed by washing out. Then the temperature increased to 95°C to 98°C to remove the remained DNA fragments from the probe, therefore from the solid surface. The DNA fragment is fractionated.

As mentioned above, the key point of this method is the occurrence of DNA polymerase reaction on DNA probe. The polymerase reaction is very sensitive to base sequence at 3' terminus of the DNA probe. When the terminal two-base sequence of the DNA probe is complementary to the corresponding sequence of DNA fragment (terminal two bases match to DNA fragment), the polymerase reaction occurs on the DNA probe. When the reverse is the case, it does not occur or hardly occurs, which results in hybrid dissociation at the high temperature of 95°C to 98°C. Consequently DNA fragments are separated according to the 3' terminus sequences and fractionated. There are sixteen combinations of two-base sequences. Many DNA fragments cannot be separated and fractionated by this hybridization and probe extension method at one time. However, the separation and fractionation of a great many DNA fragment species can be done by coupling gel electrophoresis separation with this method. The DNA fragment fractionated in this way is amplified by PCR, and is used for DNA sequencing reaction.

DNA sequencing is normally performed by gel electrophoresis. A method for sequencing long DNA using the DNA probe is disclosed in the Specification of the Japanese Patent Application No.4-180095 (Filing Date: July 7, 1992) and its corresponding U.S. Patent Application Ser. No. 08/086,892 (filed on July 7, 1993). This method uses only the presence or absence of hybridization between the DNA probe and DNA fragment for selecting primers, and fails to select the primer which is used for DNA complementary extension with a sufficient accuracy; there are cases where the 3' terminal sequence is mismatched and the probe cannot be used. In some occasions with the reported method, the length of the base on the known sequence portion is insufficient to make the stable hybrid. Furthermore, this method does not refer to DNA separation and fractionation, and is essentially different from the method of said prior art.

Said DNA separation and fractionation according to the present invention has a step of binding the oligomer having known sequence to a plurality of DNA fragments in the initial process. This step may be omitted by using double stranded oligomer of the chip.

The system used for DNA separation and fractionation according to the present invention has; i) DNA fragment fractionation subsystem comprising the vessel containing the chip wherein the oligomer which includes the known sequence and where 1 to 3 bases of the 3' terminal side is sequence-selecting is compartmentalized and is immobilized on the solid surface for each sequence selecting species, or the vessel holding the oligomer on the surface of such a solid as a bead in the chamber compartmentalized by partition walls, and ii) a robot system providing with a means of putting the solution containing the target DNA fragment into the subsystem i), and further putting DNA polymerase in it, a means of issuing the instruction to control the temperature in subsystem i) according to the specified temperature profile, a means of fractionating only the remaining DNA fragment bound to the said solid and a sample transfer means. The temperature control instruction means provided on the robot system may be mounted on the subsystem i). Furthermore, whenever required, a gel electrophoresis apparatus iii) for DNA separation and fractionation according to the length may be added to the said subsystem i) and robot ii).

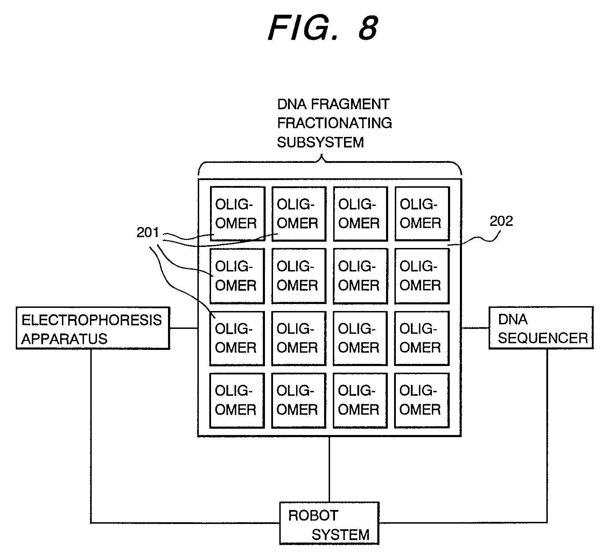

The DNA analysis system according to the present invention is a system comprising the DNA sequencer iv) added to said subsystem i) and robot ii). Whenever required, another gel electrophoresis apparatus for DNA fragment separation and fractionation iii) may be added. This gel electrophoresis apparatus iv) can be of any type if the object can be achieved. For example, the fluorescence detection type electrophoresis apparatus can be used conveniently. Fig. 8 illustrates the DNA analysis system according to the present invention. Removing the DNA sequencer removed from Fig. 8 gives a DNA separation and fractionation system according to the present invention.

### BRIEF DESCRIPTION OF THE DRAWINGS

Fig. 1 is a flow chart representing the DNA separating, fractionating and analyzing method and system according to the present invention;
Fig. 2 is a schematic representation illustrating the process of digesting the sample DNA with restriction enzyme and of introducing the oligomer having the known sequence into the terminal of the resulting DNA fragment;
Fig. 3 is a schematic representation illustrating the process of digesting the sample DNA with another restriction enzyme and of introducing the oligomer having the known sequence into the terminal of the resulting DNA fragment;
Fig. 4 is a drawing illustrating the structure of the probe immobilized on the solid surface;
Fig. 5 is a schematic representation illustrating the structure of the probe chip;
Fig. 6 is a graph representing the fluorescent intensity changing with the migration time;
Fig. 7 is a schematic representation illustrating the structure of the primer (or probe) used in the DNA fragment sequencing;
Fig. 8 is a schematic representation illustrating the configuration of the system used in DNA separating, fractionating and analyzing method according to the present invention;
Fig. 9 is a flow chart showing the analysis procedure of Embodiment 4; and
Fig. 10 is a low-separability electropherogram obtained by agarose gel electrophoresis of the DNA fragment obtained by digesting with the enzyme HhaI.

### DESCRIPTION OF THE PREFERRED EMBODIMENTS

The following gives concrete description of the present invention with reference to embodiments:

### [Example 1]

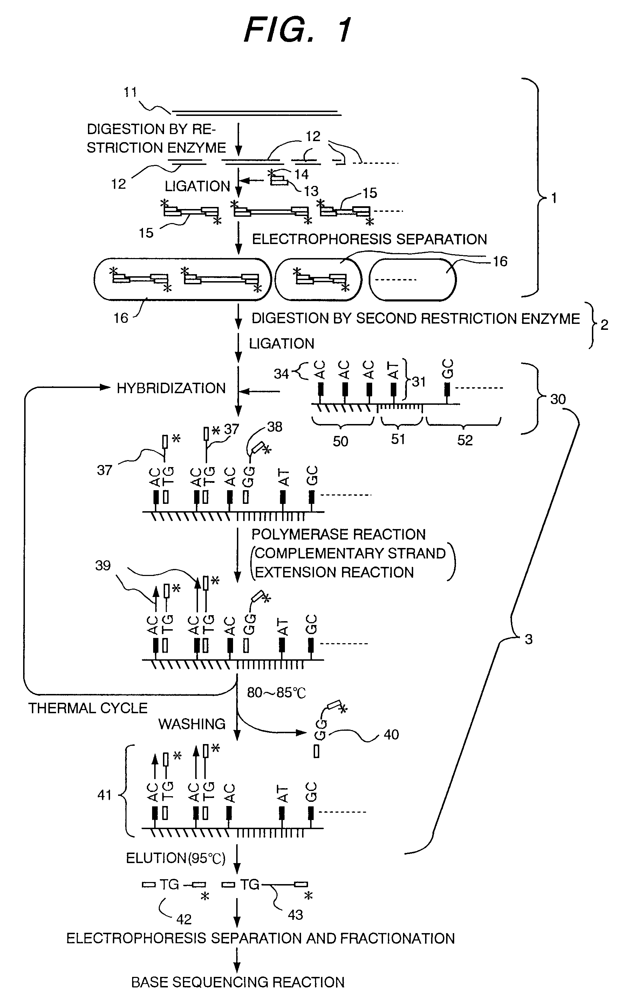

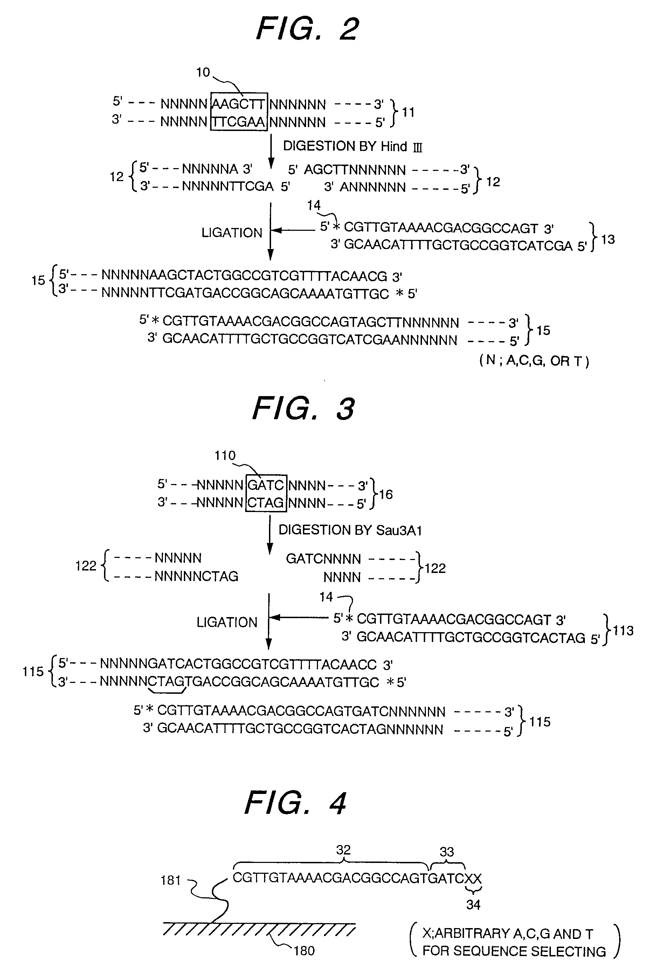

Fig. 1 is a flow chart representing the DNA analysis. The λ phase DNA has been selected as sample 11. It is digested using the 6 base recognition enzyme, for example, HindIII. Any types of restriction enzymes can be used. It includes AatII, AccI, AcyI, AflIII, AluI, ApaI, AspI, BanI, DraI, DraIII, HaeIII, EcoRI, MinfI, MaeI, NotI, PstI, HhaI, Sau3AI, ScaI, TaqI and so on. These are listed in biochemical catalogue of Biochemical company for example "Biochemicals for Molecular Biology" from Boehringer Mannheim 1990, or in "Molecular Cloning" by Sambrook, Fritsch and Maniatis, pages 5.3 to 5.9. This enzyme recognizes sequence 10 shown in Fig. 2 and digests the DNA at the illustrated position. The digested DNA fragment 12 is bound to the DNA oligomer 13 (shown in Fig. 2) having the known sequence by ligation. Use of the oligomer labeled with fluorophore 14 is effective for the following fractionation operation. The inventors used the oligomer labeled with sulforhodamine 101 (Texas Red having an emission wavelength of 620 nm). Ligation product is separated and fractionated through acrylamide gel electrophoresis. The number of DNA fragments in each fraction depends on the separability of the gel electrophoresis. When the DNA fragment having the lengthes from 2-kilo to 6-kilo bases is to be separated, fractionation is possible with the accuracy of about 1% of the length. DNA fragment species of 1 to 6 species or less can be made to be included in each fraction, as shown by 16 in Fig. 1. These DNA fragments are again digested by another restriction enzyme for each fraction, as indicated by 2 in Fig. 1. The present inventors used 4-base recognition enzyme such as Sau 3AI as restriction enzyme. The digested portion is bound with the oligomer having the known sequence by ligation. Poly A tail can be introduced into the 3' terminal by terminal transferase, but if it is digested by Sau 3A1, no recognition sequence remains at the 3' terminal of the digested product (the recognition sequence is provided on the 5' terminal). As there is no known sequence at the 3' terminus the 3' terminal of the digested portion is once extended by complementary strand extension is carried out to make the sequence complementary strand at the 3' terminus of the digested portion; then Ply A tail is attached. Fig. 3 illustrates the digested portion and known oligomer sequence. "122" denotes the digested DNA fragment. A sequence different from said known sequence 13 may be selected for this known oligomer 113. Fluorophore label 14 is attached to the oligomer to facilitate detection. "15" and "115" denote the DNA fragments where the DNA oligomers of the known sequence labeled with fluorophore are introduced. "N" in Fig. 2 means one of adenine (A), guanine (G), cytosine (C) or thymine (T).

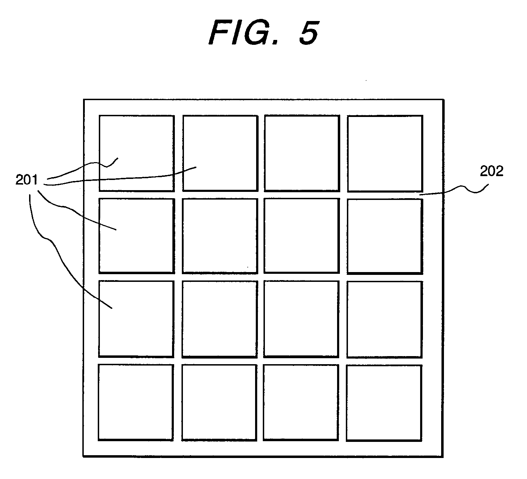

To fractionate the obtained DNA fragment, the chip 30 holding the oligomer is prepared, as shown in Fig. 1. The oligomer on chip is used to immobilize the 5' terminal side to the solid surface 180 through linker 181, as shown in Fig. 4, and contains the oligomer sequence 32 attached to the DNA fragment sample by ligation and part of restriction enzyme recognition sequence 33 on its 3' terminal side. This oligomer forms hybrid with all DNA fragments. To identity the DNA fragment species, the oligomer having further two bases for selecting sequence 34, for example, (1 to 3 bases also acceptable) on the 3' terminal side is used. Two sequence selecting bases can be selected from 16 combinations. As shown in Fig. 5, the chip surface is divided into 16 parts by means of separation wall 202, and a single species of oligomers are immobilized to each oligomer immobilization cell. This chip is used to separate and fractionate the DNA fragment. The stability of hybridization heavily depends on whether or not the complementary strand is extended by the difference of two-base sequence following the restriction enzyme recognition sequence of the DNA fragment; this finding is used for separation and fractionation. The DNA fragments are completely complementary except for two bases at the 3' terminal of the oligomer on the chip. By the degree of hybridization, it is difficult to determine whether two bases at the terminal are complementary or not. However, the complementary strand extension reaction using the DNA polymerase greatly depends on the two bases at the 3' terminus of the oligomer used as primer. Binding force between the oligomer subjected to complementary strand extension and DNA fragment is much larger than that between non-extended oligomer and DNA fragments. So after oligomer 31 immobilized at the surface of the oligomer immobilization chip 30 in Fig. 1 and the DNA fragments 37 and 38 having the known sequences have been hybridized, DNA complementary strand extension reaction is performed. Separation and fractionation are performed by obtaining only the DNA fragment linked to the strand-extended oligomer 39. The DNA fragment is put in the vessel having the chip at its bottom (the chip may be placed on the bottom of the vessel), thereby forming hybrid with the oligomer. Reference numerals 50, 51 and 52 in Fig. 1 denote oligomer immobilization areas on the chip with different oligomers immobilized. Taq DNA polymerase and complementary strand extension sequencing reaction mixture dNTP (deoxynucleotide triphospate) are added to this to promote complementary strand extension. Complementary strand extension proceeds with the hybrid 37 where two bases on the 3' terminal side of the oligomer are perfectly complementary to those of the DNA samples, and the oligomer strand immobilized on the surface extends. Complementary strand extension does not proceed with the hybrid 38 where two bases on the 3' terminal side of the oligomer are not perfectly complementary, or complementary strand extension is very slow even if it occurs. Complementary strand extension may occur even if the two bases at the terminal are not complementary. This is especially the case when these two bases comprises a combination of G and C, and one base is replaced with the other. In this case, the third base adjacent to the two terminal bases of the oligomer is replaced by inosine or mismatched base to weaken the hybridization force at the terminus, where the mismatch at the two bases is much serious for the complementary strand extension. As a result, complementary strand extension virtually takes place only when two bases at the terminal complementary to DNA fragment (match). Reaction was performed at 75 degrees Celsius which was optimum for Taq polymerase reaction. After reaction, solution temperature was raised to 80 to 85 degrees Celsius to dissociate the hybrid which had not undergo complementary strand extension. Those where two bases at the terminal which are selection sequence are not perfectly complementary are removed as in the case of DNA fragment 40. On the other hand, the hybrid subjected to complementary strand extension, such as the chip 41 having a DNA fragment held by the oligomer subjected to strand extension, is held with the extended on the solid surface. The fractionation efficiency of the DNA fragment increases by repeating this reaction cycle. For example, the probability of complete hybridization between the DNA oligomer having 16 species and specific DNA fragments in one reaction is 1/16. This means only 1/16 DNA fragment can be caught with the proper DNA oligomer for strand extension. After the second trial of the cycle reaction, one sixteen (1/16) of the residual non-trapped DNA fragments (the amount is 15/16 of the total DNA fragments) is properly hybridized with the oligomer and trapped after complementary strand extension. After repeating ten times, half of these fragments are trapped at specified areas by the extended oligomers. To fractionate only the DNAs which have been fractionated in this way, the solution temperature is raised to about 85 degrees Celsius to dissociate the hybrid which is not subjected to complementary strand extension, and the buffer solution is used for washing. The DNA fragments held in each cell are eluted at 95 degrees Celsius, and are fractionated for each cell using the capillary, etc. In some case, separation can be also performed using the gel-filled capillary, thereby ensuring more detailed separation and fractionation. Here gel electrophoresis is used; this is because different DNA fragments, with two bases at the 3' terminal being the same, may be present, as in the case of the recovered DNA fragments 42, 43 and 44. Furthermore, getting information on the length of the DNA fragment makes it possible to determine whether or not the fragment can be DNA-sequenced at one time; this will provide information helpful for sequencing. When the length of DNA fragment is 1 Kb (1000 bases long) or less, electrophoresis (separable close to 1000 bases) using long gel (50 to 60 cm) is used, or the sequences are read up to 500 to 600 bases long, and the next sequence is determined using the new primer; this method (widely known walking method) is appropriate. On the other hand, when the length exceeds 1 kb, the fragment is digested by another restriction enzyme to reduce the length, or deleting from the terminal after cloning onto the plasmid, etc. (widely known deletion method). Using such measures, the DNAs having different lengths are produced and analyzed; this method is appropriate. In DNA sequencing, the analysis method differs according to the length of the relevant DNA fragment, so it is important to identify the DNA fragment length for gel electrophoresis. For separation and fractionation by gel electrophoresis, the prevent inventors used a columnar gel (capillary gel : 4%) generated within a glass capillary having an inner diameter of 0.3 mn and length of 20 cm, fractionated the DNA while monitoring the DNA melting from the capillary bottom by means of a fluorescence detection type electrophoresis apparatus. Two bases leading to the restriction enzyme recognition sequence on the 3' terminal side of the DNA fragment contained in each fraction are already known; therefore, the PCR amplification was made using the primer having at the 3' terminal the sequence complementary with these two bases in addition to the common portion of the ligated oligomer or of poly A tail and the primer having only sequence complementary to the known common portion sequence (part of the oligomer added by ligation). The number of double strand DNA segment copies was increased for the use as sequencing template, and analysis was made.

Reference numeral 1 in Fig. 1 denotes the process of fragmentation and approximate separation of the sample DNA by the first restriction enzyme, and 2 shows the process of re-fragmentation by the second restriction enzyme, while 3 denotes the process of separating the DNA fragment by oligomer immobilization chip.

In the present example, electrophoresis was conducted after fractionating various DNA fragments for each terminal sequence using the chip holding the oligomer. This order may be reversed.

Namely, the DNA fragment group is separated according to the length by gel electrophoresis. When the DNA fragment is 1 Kb long or less, the accuracy of separation according to length is 1 to 2 base. In the case of the DNA fragment mixture, it is easy to use 2 to 6 species for each fraction. (The DNA fragment is handled as two stands, so two species of DNA fractionation are the minimum.) The terminal two bases (two bases following the restriction enzyme digestion sequence) of the DNA fragments in each fraction was subjected to DNA complementary strand extension by using the 16 species of oligomers. The terminal base was identified and fractionated according to the presence or absence of complementary strand extension, and PCR amplification was conducted to provide samples for DNA sequencing.

Fig. 8 illustrates an example of the DNA analysis system configuration used in the present embodiment.

### [Example 2]

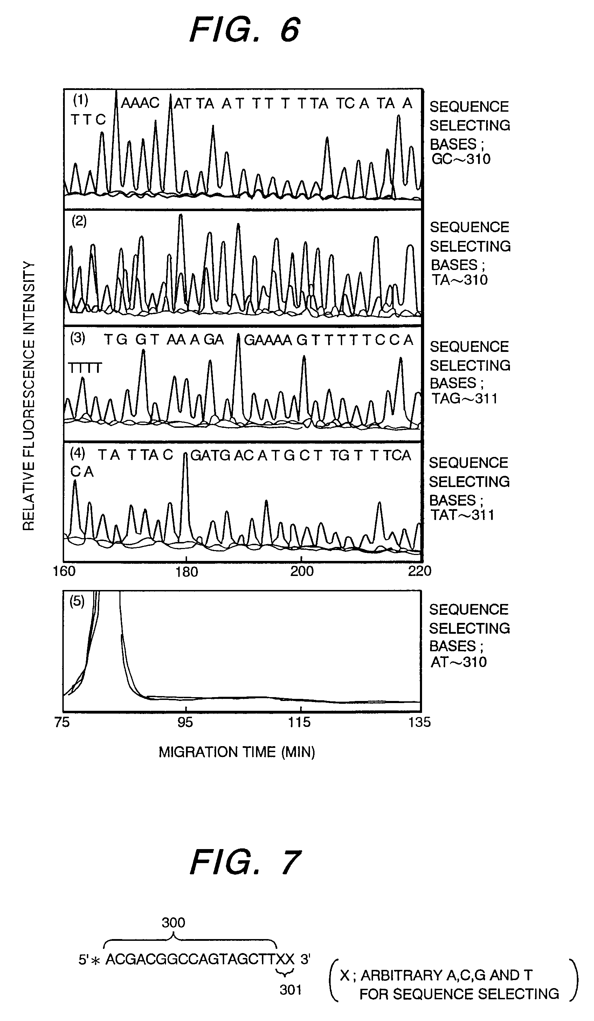

Fig. 6 is a graph representing the DNA fragment spectrum as an example of base sequencing. It shows the result of sequencing the fragment obtained by digesting the M13 phage DNA with Hind III and Sau3A1. As shown in Fig. 7, the sequencing primer has the sequence complementary to the part of the common sequence 300 and two bases at the terminal for selecting DNA fragment 301. If the primer with mismatched two bases at the terminal is used, complementary strand extension is not carried out at all, so sequence information cannot be obtained (Fig. 6 -(5)). If the primer match with one species of DNA fragment alone in the fraction, the spectrum shown in fig. 6-(1) is gained by base sequencing reaction using the matched primer having two-base selective sequence. It shows that the primer is used successfully for selecting a fragment from a mixture 310 by the sequence difference at the 3' terminal. DNA sequencer based on gel electrophoresis is used to this ensure sequencing. On the other hand, when electrophoresis separation of DNA fragments is not performed and, or therefore, there are many fragments in the fraction, it is highly probable that two species or more of the DNA fragments having the same terminal 2-base sequence are included in one fraction. For example, when two species of DNA fragments which match one primer are contained, the peaks attributable to two species of DNA fragments in the sequencing spectrum gained with one primer as shown in Fig. 6-(2) are overlapped with each other, and this makes things complicated. The sequence is determined using the primer 311 having the three-base sequence used for selecting a fragment from a mixture on the 3' terminal, and DNA sequencing reaction is performed; then information on individual base sequence can thereby be obtained (Fig. 6 - (3) and (4)). If long DNAs having several tens of kilo bases or longer are digested into a short fragments as several hundreds bases, many fragment species will be formed; this will take much time. So without producing many fragment species at one time, the DNA is digested by the 6-base recognition enzyme, which makes 4 kilo base fragments on average, and is fractionated by electrophoresis. Then it is digested for each fragment by the 4base recognition enzyme to be made to have the size which allows analysis. Then it is fractionated by gel and oligo-chip; this method is preferable.

### [Example 3]

The present embodiment represents a case where the number of original DNA copies is small. The DNA is fragmented by enzyme or the like, and oligomer is bound by ligation. The fragments are fractionated according to their length by gel electrophoresis. Several species of DNA fragments are included in one fraction. They are put into the vessel to the bottom of which the oligomers used for fractionating DNA fragments are immobilized. They are subjected to complementary strand extension after hybridization by DNA fragment. Then the temperature is raised to 80 degrees Celsius to dissociate the hybrid which has failed to undergo complementary strand extension. After lowering the temperature, complementary strand extension is repeated. After repeating said process several times, add the primer having the same sequence as that of the oligomer used for ligation, and PCR amplification takes place. Amplification occurs in the solution and on the solid surface, but a little reduction in the amount of the added primer will increase the percentage of complementary strand extension on the solid surface. Furthermore, if the oligomers of different sequences are bound to both terminals of the DNA fragment, only the PCR amplification on the solid surface occurs. That is, the primer having oligomer sequence of one side is immobilized on the solid surface, and the oligomer having the same sequence as that of the oligomer bound to another DNA terminal is put into solution. PCR amplification takes place only after both primers are made ready; therefore, DNA fragment amplification occurs only on the solid surface.

The plate-formed solid surface has been used in the present Embodiment. It is possible to use the bead, capillary or porous solid to increase the surface area so that the number of the oligomers to be held is increased.

As discussed above, when the amplification of DNA fragment occurs only on the solid surface, PCR is performed with the oligomers immobilized on the solid surface to amplify the required DNAs and to pick them up. This makes it possible to increase different DNAs simultaneously for each fraction under the separated state. This is not only convenient for fractionation but also saves the reagent.

In the present Example, the DNA was digested by restriction enzyme, and the oligomer of the known sequence was bound to it by ligation. Or poly A tail was formed at the 3' terminal by terminal transferase and was used. It should be noted that Abases can be added to the 3' terminal of the single strand DNA strand one after another by the terminal transferase.

As an alternative to this method, the following method may be used: The DNA is digested at random by supersonic or the like, and poly A tail is formed at its 3' terminal by terminal transferase. The sequence of several bases adjacent to this poly A tail is used as selection sequence, thereby fractionating the specific DNA. In this case, it is difficult to get the single species of the DNA fragment in the first selection, so selection should be repeated several times. That is, if selection is made by the two bases (e.g. GG) of the terminal for the first occasion, then selection is made by the terminal four-base (e.g. GAGG) including this. For the third time, selection is made by terminal six bases including these four bases. Selection is repeated in this way. Selectivity of the specific strand selection by complementary strand extension depends most heavily on the degree of matching of hybridizations of terminal 1 and 2 bases. Repetition of the selection of terminal two bases is effective to improve fractionation accuracy.

Furthermore, biotin label is put in the oligomer, and is made to hybridize with DNA. After complementary strand extension is over, biotin-avidin bond is formed with the avidin immobilized at the solid layer; thereby it is trapped by the solid layer.

### [Example 4]

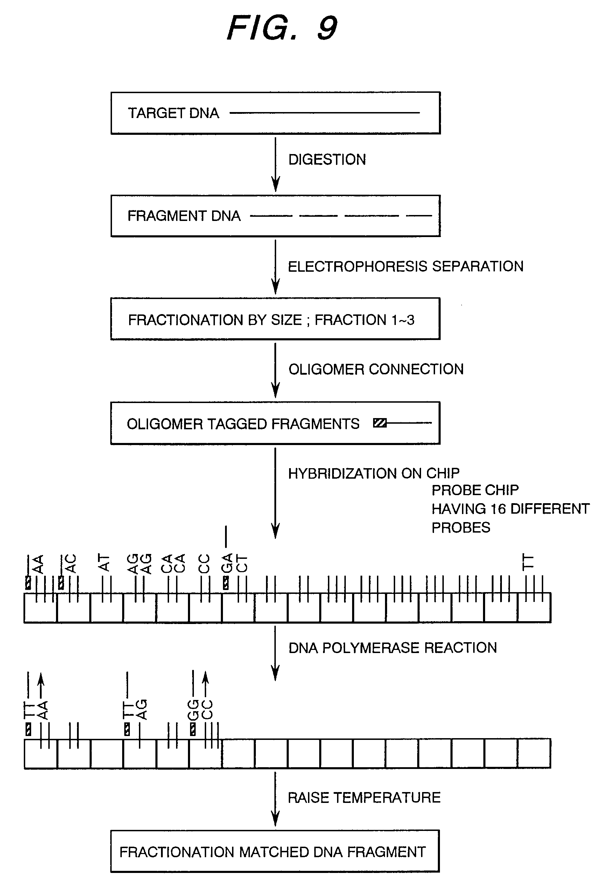

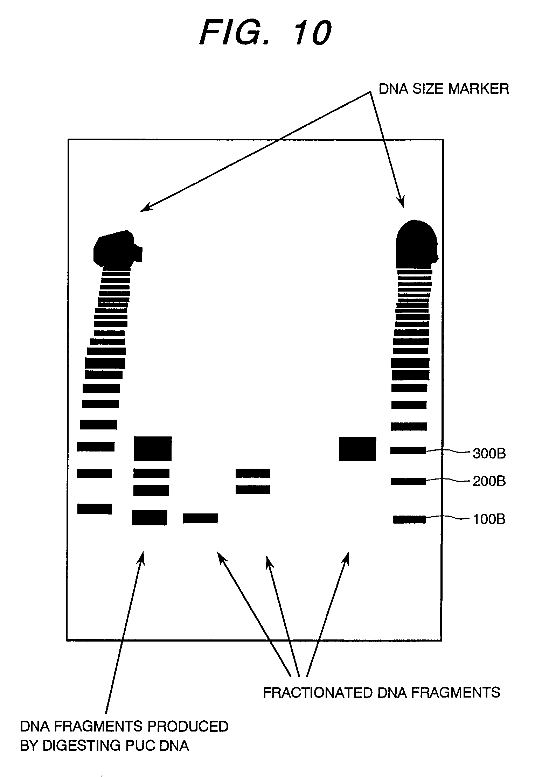

PUC DNA was selected as the sample DNA for DNA sequencing. The DNA size of PUCD is about 2.7 kilo bases which is too long to be sequenced at one time. According to this invention, DNA is digested by enzyme to small pieces which can be sequenced by single operation. Many fragment species are produced, which are sequenced in parallel using a library of the 16 primers (two-base-selective primers). Fig. 9 illustrates the flow of this Embodiment. DNA is digested by restriction enzyme HhaI (digested as e.g. 
5' ... GCG|C ... 3'
3' ... C|GC G ... 5'),
and the resulting fragments are fractionated by gel electrophoresis. Of course, any types of restriction enzymes can be used. Fig. 10 shows an electrophoresis pattern. It was broadly classified into three groups, which were fractionated (faction 1 to faction 3). Then the oligomer is introduced into the terminal. Introduction is made by bonding the oligomer with DNA fragment by ligase which is DNA modification enzyme, or by attaching the poly A tail to the 3' terminal of DNA fragment using use of terminal transferase. According to the ligase method, the length of the oligomer to be bonded is constant, and fragments of a constant length are produced in conformity to the original length. So this operation may be performed prior to electrophoresis. In poly A tail method, on the other hand, the lengths of the poly As are different in each use due to terminal transferase activity. At the time of separation by electrophoresis, and the band will expand and accurate separation length cannot be obtained. So the DNA fragment is separated by gel electrophoresis; then the poly A tail is attached. A plurality of species of DNA fragments are included in factions 1 to 3. They are analyzed by the selective DNA probe or are fractionated for each DNA fragment.

The selective DNA probe is of course used as the primer for polymerase reaction, therefore, DNA sequencing. A primer library consisting the primers which have the sequence complementary to the introduced sequence, a part of recognition sequence (X₁-X₂ ... Xₙ; n=2-6) of the restriction enzyme and the selective sequence (Y₁ .. Yₘ; m=1-3). The primer can be represented as 5' ... X₁..Xₙ·Y₁..Yₘ3'. The characteristic of the primers in a library is having 1 to 3 bases of every sequences at 3' termini for selecting DNA fragments and a part of recognition sequence adjacent to the introduced sequence. The sequence X₁ .. Xₙ recognize the fragment terminus when the primer hybridize with the fragment. The introduced sequence makes the hybrid stable at least during the polymerase reaction. The selective sequence of Y₁ .. Yₘ distinguishes the fragments by promoting or inhibiting the polymerase reaction according to the matching with the fragment sequence. The enzyme Pst I recognizes the sequence 
C TGCA G
G ACGT C
and cut it at ↓ position. When PstI is used, the sequence X₁ .. Xₙ becomes TGCAG. When HhaI (which digest 
G CG C )
C GC G
is used, it becomes CGC. In the selective sequence, Y₁, .. Yₘ can take any of A, C, G, or T. When m = 1, Y₁ = A, C, G, or T and 4 primers are in the library. When m = 2, Y₁ · Y₂ = AA, AC, AG, AT, CA, CG, ... TT and 16 primers are in the library. Similarly, when m = 3, 64 primers are in the library.

The library can be prepared for each restriction enzyme. Practically, the primer libraries for 3 to 4 enzymes are enough to sequence various DNA fragments. The primer library of 16 primers which have two-base selective sequences is most effective because the number of primers is not to large and the selection of fragments is carried out efficiently.

In the first example, the terminal 2-base selection primer was used to determine the working primer. It shows two cases; the case of sequencing directly from the mixture and the case of sequencing after fractionation. Part of each faction is picked up and is divided into 16 equal parts. Selective DNA probe Tag polymerase and deoxy nucleotide triphosphate (dNTP) are added to each of them, thereby causing DNA polymerase reaction to take place. The selective DNA probe is a fluorophore-labeled primer having biotin and sulforhodamine 101 close to the 5' terminal, and its the sequence is any of the 5' TT ... TCGCXY(X, Y: A, C, G, or T). Only fractions containing the matched primer produce the extended primer as the reaction product. By analyzing the length of the product by gel electrophoresis, the primers which undergo perfect hybridization with the DNA fragments (match) and the length of the fragments are clarified. Then the hybrids between these DNA probes and DNA fragments are picked up by the bead holding avidin on the surface and fractionated. Of course, this picking up process can be omitted for sequencing because DNA sequencing directly from the fragment mixture is possible.

DNA sequencing is done by preparing the DNA sample according to the normal protocol using the primer having the same sequence as that of the used DNA probe. The DNA fragment serving as a template for sequencing may be in a mixed state, but better results can be obtained by using it after picking it up by the bead. The sequence of each fragment is determined as stated above with a small library of 16 primers. The whole sequence is constructed by connecting each sequence with the information of adjacent sequences of the cutting site of fragments. In the occasion, the fragments digested by other enzyme are sequenced and used for the connections.

In anycase, this process does not require the cloning process, nor a library of many primers as several hundreds or thousands required for primer walking with a library of primers.

As discussed above, according to the present invention, a combination between the separation by the length of the DNA fragment and separation by DNA terminal sequence allows easy DNA sequencing with separated primers and a single DNA fragment to be obtained without cloning. A combination with the PCR permits the long DNA to be fragmented without cloning process, and allows the fragments to be separated, thereby increasing the number of copies to carry out analysis for base sequencing.

## Claims

1. DNA separation and fractionation method comprising:
i) a process of preparing the chip wherein the DNA probe comprising the oligomer which includes the sequence common to all the probes and selective sequence of 1 to 3 bases at the 3' terminal which is used to select the fragment is immobilized on the solid surface for each selective sequence species, or a process of holding the probe in a compartmentalized vessel;
ii) a process of hybridizing a plurality of DNA fragments to said DNA probe;
iii) a process of stabilizing hybrid between the DNA probe and said DNA fragment by extending only the DNA probe in which the selective sequence on the 3' terminal side of DNA probe following the known sequence is complementary to the sequence portion to which said DNA fragment corresponds through DNA polymerase reaction; and
(iv) a process of separating and fractionating the specific DNA fragment by the difference of hybrid stability through heating treatment following the processes iii).

2. A DNA separation and fractionation method comprising:
i) a process of binding the oligomer having known sequence to a plurality of DNA fragments at the 3' termini;
ii) a process of preparing the solid such as a chip or bead wherein the DNA probe comprising the oligomer which includes the complementary sequence with the known sequence and the base selective sequence comprising 1 to 3 bases of the 3' terminal side is immobilized on the solid surface individually for each sequence selecting species, or a process of holding it in a compartmentalized vessel;
iii) a processing of hybridizing the DNA fragment obtained in process i) to the oligomer on said solid, using the solid holding the DNA probe obtained in process ii);
iv) a process of stabilizing hybrid between the DNA probe and the DNA fragment obtained in process i) by extending only the DNA probe in which the selective sequence on the 3' terminal side of DNA probe following the sequence complementary to the known sequence; and
(v) a process of separating and fractionating the specific DNA fragment by the difference of hybrid stability during heating treatment following the process iv).

3. A DNA analysis method comprising a process of analyzing the DNA sequence of the DNA fragment separated and fractionated by amplifying, or without amplifying, the number of copies of the specific DNA fragments separated and fractionated after completion of all the processes of Claim 2.

4. A DNA analysis method according to Claim 1 comprising a process i') of preparing said plurality of DNA fragments by digesting the target DNA sequence with the restriction enzyme prior to process ii).

5. A DNA analysis method comprising a process of analyzing the DNA sequence of the DNA fragment separated and fractionated by amplifying, or without amplifying, the number of copies of the specific DNA fragments separated and fractionated after completion of all the processes according to Claim 4.

6. A DNA separation and fractionation method according to Claim 2 further comprising:
v') a process of dissociating the hybrid which was not subjected to complementary strand extension by heating the hybrid between the DNA fragment obtained in process 1 and said DNA probe on the solid layer, said process taking place of process v) after completion of process iv);
vi) a process of repeating processes iii), iv) and v') by reducing the temperature of hybrid and DNA fragment obtained in process v'), and the mixtures of the DNA probes on the solid layer which are not hybridized; and
vii) a process of amplifying the specific DNA fragments by PCR and selecting and fractionating them by causing the oligomer of the sequence complementary to the known sequence used in process to be added to the mixtures after having gone through process iv).

7. A DNA separation and fractionation method according to Claim 1 comprising a process of separating and fractionating the DNA fragment by gel electrophoresis before process ii) or after process iv).

8. A DNA separation and fractionation method according to Claim 2 comprising a process of separating and fractionating the DNA fragment by gel electrophoresis before process iii) or after process v).

9. A DNA separation and fractionation method according to Claim 2 wherein said DNA probe in process 1 is fluorophore-labeled.

10. A DNA analysis method according to Claim 3 or claim 5, wherein the process of analyzing said DNA sequence is a process of gel electrophoresis.

11. A DNA separation and fractionation system comprising:
i) DNA fragment fractionation subsystem comprising the vessel containing the chip wherein the oligomer which includes the known sequence and where 1 to 3 bases of the 3' terminal side is selective sequence is compartmentalized and is immobilized on the solid surface for each selective sequence species, or the vessel holding the oligomer on the surface of such a solid as a bead in a compartmentalized chamber; and
ii) a robot system providing with a means of putting the solution containing the target DNA fragment into the subsystem i), and further putting DNA polymerase in it, a means of issuing the instruction to control the temperature in subsystem i) according to the specified temperature profile, and a means of fractionating only the remaining DNA fragment bound to the said solid and a sample transfer means.

12. A DNA separation and fractionation system according to Claim 11 further comprising:
iii) a gel electrophoresis apparatus for separation and fractionation the length of the DNA fragment.

13. A DNA analysis system comprising the DNA separation and fractionation system of Claim 11 or Claim 12 and further comprising:
iv) a DNA sequencing apparatus.

14. A DNA analysis system according to Claim 13 wherein said DNA sequencing apparatus is a gel electrophoresis apparatus.

15. A DNA analysis method comprising:
i) a process introducing the known sequence to the target DNA fragments at their 3' termini:
ii) a process of preparing a primer library comprising at least 16 primers and less than 64 primers for each restiction enzyme which include the sequence complementary to the introduced known sequence at the target DNA fragment and a part of recognition sequence of the restriction enzyme and the selective sequence of 1-3 bases at 3' termini;
iii) a process of hybridizing the probe with the fragment to perform DNA sequencing.

16. A sequencing primer library consisting at least 4 primers which have sequences of 5' ... X₁ ... Xₙ·Y₁3', where X₁ ∼ Xₙ (n = 2 ∼ 6) represent a part of recognition sequence of restriction enzyme, and Y₁ is the selective base of A, C, G or T.

17. A sequencing primer library consisting at least 16 primers which have sequences of 5' ... X₁ .. Xₙ·Y₁Y₂3', where X₁ ∼ Xₙ (n = 2 ∼ 6) represent a part of recognition sequence of restriction enzyme, and Y₁Y₂ (Y₁, Y₂ = A, C, G, or T) is the selective sequence.

18. A sequencing primer library consisting at least 64 primers which have sequences of 5' ... X₁.. Xₙ·Y₁Y₂Y₃3', where X₁ ∼ Xₙ (n = 2 ∼ 6) represent a part of recognition sequence of restriction enzyme, and Y₁Y₂Y₃ (Y₁, Y₂, Y₃ = A, C, G, or T) is the selective sequence.

19. A sequencing primer library consisting at least 16 primers which have sequences of 5' ... X₁ ... XₙZ₁..ZₘY₁Y₂3', where X₁ ∼ Xₙ (n = 2 ∼ 6) represent a part of recognition sequence of restriction enzyme, Z₁∼ Zₘ (m = 1 ∼ 4) represent the nucleotide analogue, and Y₁ Y₂ (Y₁, Y₂ = A, C, G, or T) is the selective sequence.

20. A DNA analysis method comprising:
i) a process of creating an area for DNA probe to hybridize by introducing the oligomer having the known sequence into the terminals of these DNA fragment by addition of bases by ligation or terminal transferase, using the DNA sample containing a plurality of DNA fragment species;
ii) a process of hybridizing a plurality of DNA fragments with DNA probes wherein the DNA probes for the DNA fragments selection which include the sequence complementary to said known sequence and 1 to 3 bases on the 3' terminal side are sequence-giving are compartmentalized and immobilized on the chip or wherein probes are held in the compartmentalized vessel;
iii) a process of boosting hybridization between the DNA probe and said DNA fragment by extending only the DNA probe in which the sequence for selection on the 3' terminal side following the known sequence is perfectly complementary to the sequence to which said DNA fragment corresponds, out of the DNA probes hybridized in process ii) by complementary strand extension through DNA polymerase; and
iv) a process of identifying the probe subjected to complementary strand extension in process iii) and conducting DNA sequence analysis based on Sanger's method using the primer having basically the same sequence, or of fractionating and analyzing the DNA fragment hybridized with the DNA probe subjected to complementary strand extension.
